Europäisches Patentamt

European Patent Office

Office européen des brevets

(19)

(11) Publication number: **0 146 039**
**A2**

(12) # EUROPEAN PATENT APPLICATION

(21) Application number: **84114443.9**

(22) Date of filing: **29.11.84**

(51) Int. Cl.⁴: **C 12 Q 1/68**

---

(30) Priority: **12.12.83 US 560429**
**31.08.84 US 645850**
**07.11.84 US 668256**

(43) Date of publication of application: **26.06.85**
**Bulletin 85/26**

(84) Designated Contracting States: **AT BE CH DE FR GB IT LI LU NL SE**

(71) Applicant: **MILES LABORATORIES, INC., 1127 Myrtle Street, Elkhart Indiana 46514 (US)**

(72) Inventor: **Albarella, James P., 25845 Meadow Oak Lane, Elkhart IN 46514 (US)**
Inventor: **Anderson DeRiemer, Leslie H., 634 Hollyridge Dr., Encinitas CA 92024 (US)**
Inventor: **Carrico, Robert J., 54256 Silver Street, Elkhart IN 46514 (US)**

(74) Representative: **Adrian, Albert, Dr. et al, c/o BAYER AG Konzernverwaltung RP Patentabteilung, D-5090 Leverkusen 1, Bayerwerk (DE)**

---

(54) **Hybridization assay with immobilization of hybrids by antihybrid binding.**

(57) A nucleic acid hybridization assay wherein the hybrid formed between the polynucleotide sequence to be detected and the probe is immobilized by binding of an immobilized or immobilizable form of an antibody reagent selective for binding such hybrid. Preferred antibody reagents are selective for DNA·RNA or RNA·RNA hybrids or for intercalated duplexes. The immobilized hybrid is detected preferably by use of a labeled form of a second such antibody reagent or a labeled form of the probe. The method dispenses with the need to immobilize either the probe or sample nucleic acids and permits hybridization to proceed between the sequence to be detected and the probe in solution, resulting in rapid rates of hybridization.

## HYBRIDIZATION ASSAY WITH IMMOBILIZATION
## OF HYBRIDS BY ANTI-HYBRID BINDING

### FIELD OF THE INVENTION

This invention relates to nucleic acid hybridization assay methods and reagent systems for detecting specific polynucleotide sequences. The principle of nucleic acid hybridization assays was developed by workers in the recombinant DNA field as a means for determining and isolating particular polynucleotide base sequences of interest. It was found that single stranded nucleic acids, e.g., DNA and RNA, such as obtained by denaturing their double stranded forms, will hybridize or recombine under appropriate conditions with complementary single stranded nucleic acids. By labeling such complementary probe nucleic acids with some readily detectable chemical group, it was then made possible to detect the presence of any polynucleotide sequence of interest in a test medium containing sample nucleic acids in single stranded form.

In addition to the recombinant DNA field, the analytical hybridization technique can be applied to the detection of polynucleotides of importance in the fields of human and veterinary medicine, agriculture, and food science, among others. In particular, the technique can be used to detect and identify etiological agents such as bacteria and viruses, to screen bacteria for antibiotic resistance, to aid in the diagnosis of genetic disorders such as sickle cell anemia and thalassemia, and to detect cancerous cells. A general review of the technique and its present and future significance is provided in Biotechnology (August 1983), pp. 471-478.

## INFORMATION DISCLOSURE

The following information is provided for the purpose of making known information believed by the applicant to be of possible relevance to the present invention. No admission is necessarily intended, nor should be construed, that any of the following information constitutes prior art against the present invention.

The state-of-the-art nucleic acid hybridization assay techniques generally involve immobilization of the sample nucleic acid on a solid support. Hybridization between particular base sequences or genes of interest in the sample nucleic acids and a labeled form of the probe nucleic acid is determined by separating the solid support from the remainder of the reaction mixture which contains the unbound labeled probe, followed by detection of the label on the solid support.

The need to immobilize sample nucleic acids in order to conduct the state-of-the-art hybridization assay poses two significant problems. Firstly, the procedures required to accomplish immobilization are generally time consuming and add a step which

MS-1362

is undesirable for routine use of the technique in a clinical laboratory. Secondly, proteins and other materials in the heterogeneous sample, particularly in the case of clinical samples, can interfere with the immobilization of the nucleic acids.

As alternatives to immobilizing sample nucleic acids and adding labeled probe, one can use an immobilized probe and label the sample nucleic acids *in situ*, or one can use a dual hybridization technique requiring two probes, one of which is immobilized and the other labeled [Methods in Enzymology 65:468(1968) and Gene 21:77-86(1983)]. The former alternative, however, is even less desirable since the *in situ* labeling of the sample nucleic acids requires a high degree of technical skill which is not routinely found in clinical technicians and there are no simple, reliable methods for monitoring the labeling yield, which can be a significant problem if the labeling media contain variable amounts of inhibitors of the labeling reaction. The dual hybridization technique has the disadvantages of requiring an additional reagent and incubation step and the kinetics of the hybridization reaction can be slow and inefficient. The accuracy of the assay can also be variable if the complementarity of the two probes with the sample sequence is variable.

The use of immobilized RNA probes and detection of resulting immobilized DNA˙RNA or RNA˙RNA hybrids by labeled antibodies selective for such respective hybrids is described in commonly assigned U.S. Patent Application Serial No. 616,132, filed June 1, 1984. This method eliminates the need to immobilize or label sample nucleic acids, however, the probe strand involved in hybridization is immobilized which can significantly decrease the rate at which the probe and the sequence of interest can reanneal.

Techniques for directly detecting the polynucleotide duplex formed as the product of hybridization between the sample and probe polynucleotides, and thereby dispensing with the chemical labeling and immobilization of sample or probe polynucleotides, have been generally unsatisfactory. Attempts to generate antibodies which will selectively bind double stranded DNA·DNA hybrids over single stranded DNA have failed [Parker and Halloran, "Nucleic Acids in Immunology", ed. Plescia and Braun, Springer-Verlag, NY (1969) pp. 18 *et seq*]. Some success has been achieved in generating antibodies that will bind RNA·DNA mixed hybrids or RNA·RNA hybrids and have low affinity for the single stranded polynucleotides [see, for example, Rudkin and Stollar, Nature 265:472(1977)]. However, these methods are described, as in the case of the hybridization techniques discussed above employing labeled probes, as requiring immobilization of the sample nucleic acids. Rudkin and Stollar fixed whole cells on microscope slides and exposed the DNA in the nucleus. It was hybridized with an RNA probe and the hybrid was detected by fluorescence microscopy with fluorescein-labeled antibody to RNA·DNA.

Early procedures for detection of specific DNA sequences followed solution-phase formats wherein hybridization involved soluble forms of both the

MS-1362

sample DNA and a radiolabeled RNA probe. The hybrid formed in the solution was isolated by density-gradient centrifugation [Hall and Spiegelmann (1961) Proc. Natl. Acad. Sci. 47:137]. The centrifugation was too slow and impractical for analysis of large numbers of samples. This solution hybridization method was made more practical by the discovery that nitrocellulose filters strongly adsorb single-stranded DNA along with any hybridized RNA [Nygaard and Hall (1964) J. Mol. Biol. 9:125]. The labeled RNA probe binds very weakly to the filters and the method was improved by exposing the filters to ribonuclease which hydrolyzes the single stranded probe but not the RNA·DNA hybrid. The method is limited to hybridizations of DNA samples with RNA probes and the digestion with ribonuclease has to be controlled carefully; therefore, solid-phase hybridization methods became more important.

Accordingly, there is a great need for a nucleic acid hybridization assay which does not require the immobilization of either probe or sample nucleic acids and which overcomes the limitations of the known solution-phase hybridization techniques. Further, such technique should allow the use of a variety of labels, particularly of the nonradioisotopic type. A nucleic acid hybridization assay method and reagent system having these and other advantages are principal objectives of the present invention.

## SUMMARY OF THE INVENTION

The present invention provides a nucleic acid hybridization assay wherein the hybrid formed

- 6 -                                    0146039

between the particular polynucleotide sequence to be detected and a nucleic acid probe becomes immobilized by binding to an immobilized or immobilizable form of an antibody reagent selective for binding to such hybrids.. According to preferred embodiments, this permits separation of hybridized and unhybridized probe if a labeled probe is employed or separation of hybrid-bound from free labeled second antibody reagent if a labeled form of anti-hybrid reagent is used. The present method thus dispenses completely with the need for immobilization of either sample or probe nucleic acids and allows hybridization to proceed in solution where the rate of hybridization is rapid and more efficient.

In general, the method of the present invention comprises contact of a test sample containing single stranded nucleic acids with the probe which has a single stranded base sequence that is substantially complementary to the sequence to be detected. This hybridization step will proceed under conditions that are favorable to hybridization between the probe and the sequence to be detected. The resulting hybrids can then be detected by addition of the antibody reagent which is selective for binding duplexes formed by hybridization between the complementary probe sequence and the sequence to be detected. At this stage of the assay, the antibody reagent will normally be in an immobilized state, such as being fixed chemically or physically to a solid support. Alternatively, the anti-hybrid can be contacted

MS-1362

with the formed hybrids in a soluble form and thereafter rendered immobilized such as by contact with an immobilized form of an antibody raised against the anti-hybrid or by employing a ligand-modified anti-hybrid and addition of an immobilized form of a binding partner for the ligand.

The resulting duplexes that become bound to the immobilized antibody reagent can be determined in a variety of manners. Normally, one will use a labeled probe or a labeled form of a second anti-hybrid reagent. In the first instance, hybridized labeled probe that becomes associated with the immobilized phase is separated from unhybridized probe that remains in solution and either the label associated with the immobilized phase or the label in the remaining unhybridized probe is measured. Where a labeled anti-hybrid is used, that which becomes associated with the immobilized phase by binding to formed hybrid is separated from that which remains in solution and again the label that either has become associated with the immobile phase or remains with unbound labeled anti-hybrid is measured. A variety of labels can be used including radiolabels, and preferably, nonradioisotopic labels such as enzymes, fluorescers, ligands, and the like.

The present invention is characterized by a number of significant advantages.

First, the hybridization of sample nucleic acids with a probe can be conducted in solution where the reaction rates are fastest. Typical solid-phase hybridization rates are limited by diffusion of the labeled probe to the sample

MS-1362

nucleic acid immobilized on the solid surface. This limitation does not occur with solution hybridization.

Second, nonspecific binding of a labeled probe to the solid phase used to immobilize the sample nucleic acid is a problem in solid-phase methods. Typically, the sample nucleic acids are adsorbed onto the solid phase; therefore, the solid must be a substance which binds nucleic acids with high affinity, but this property becomes a problem in the subsequent hybridization step where nonspecific adsorption of the labeled probe is to be minimized. In the present invention, the solid-phase for anti-hybrid immobilization can be chosen from materials which do not adsorb nucleic acids.

Also, most hybridization methods require immobilization of sample nucleic acids by adsorption to a solid surface or by covalent coupling to a solid. Proteins and other materials in the sample interfere with the immobilization; therefore, the sample has to be purified by methods such as phenol extraction. The present solution hybridization method immobilizes the hybrids with antibodies which have high affinity and specificity for the hybrid and are not subject to interference by proteins, carbohydrates and lipids in the sample.

Further, when sample nucleic acids are immobilized by adsorption or covalent coupling, there is no convenient means to insure that the nucleic acids are immobilized in high and reproducible yields. Furthermore, the adsorption process makes part of the polynucleotide sequence unavailable for hybridization. For instance, less than half of the DNA adsorbed onto a nitrocellulose membrane is available for hybridization. Thus, the detection limits of the assay are not optimal and the yield of hybrid can be variable. Hybridization in solution as permitted by the present invention allows the sample nucleic acids to form hybrids with maximum efficiency.

BRIEF DESCRIPTION OF THE DRAWINGS

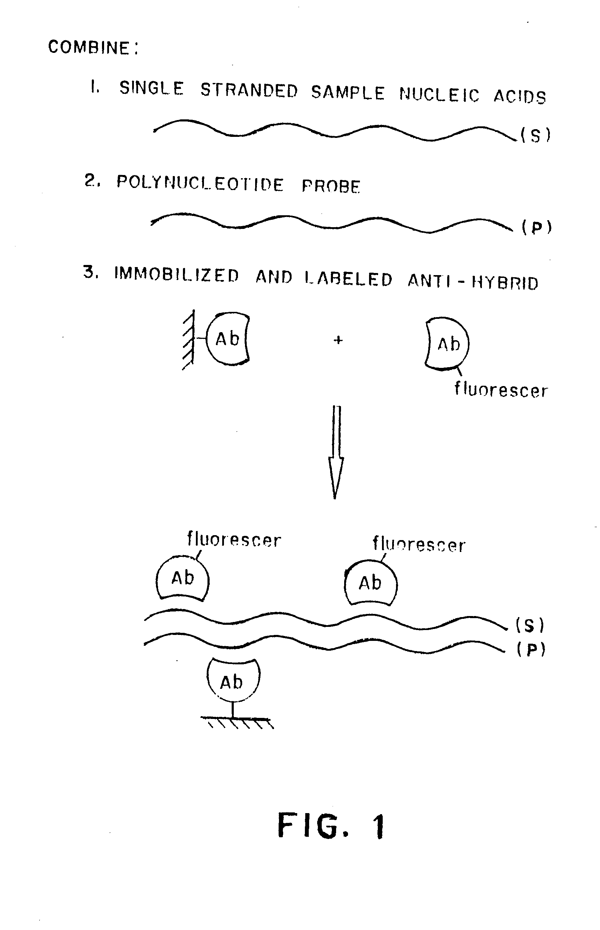

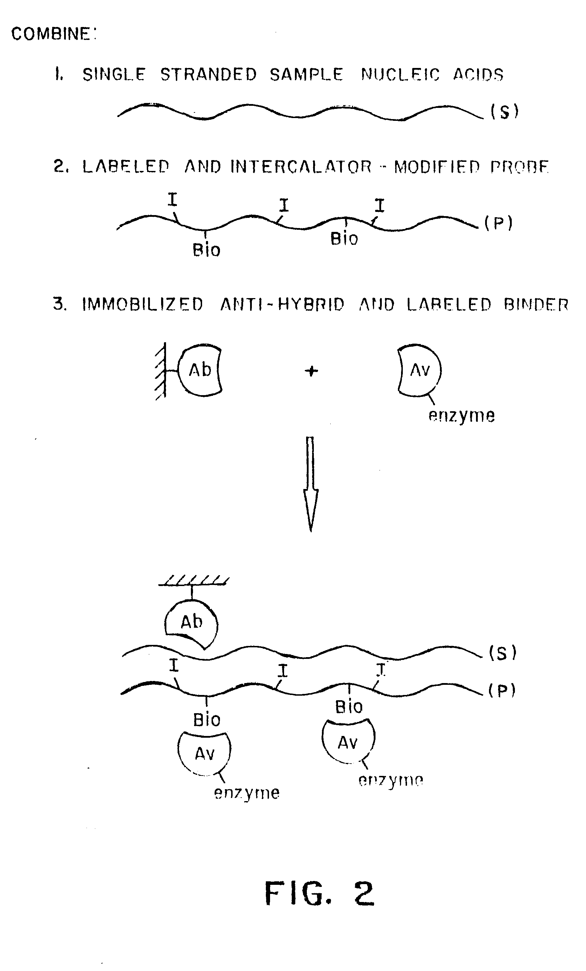

Figs. 1 and 2 are schematic illustrations of preferred methods for performing the present invention. These methods are described in detail below.

DESCRIPTION OF THE PREFERRED EMBODIMENTS

The use of nucleic acid hybridization as an analytical tool is based fundamentally on the double-stranded, duplex structure of DNA. The hydrogen bonds between the purine and pyrimidine bases of the respective strands in double-stranded DNA can be reversibly broken. The two complementary single strands of DNA resulting from this "melting" or "denaturation" will reassociate (sometimes referred to as reannealing or hybridization) to reform the duplex structure. As is now well known in the art, contact of a first single stranded nucleic acid, either DNA or RNA, which comprises a base sequence sufficiently complementary to (i.e., "homologous with") a second single stranded nucleic acid under appropriate conditions, will result in the formation of DNA·DNA, RNA·DNA, or RNA·RNA hybrids, as the case
may be.

*The Probe*

The probe will comprise at least one single
stranded base sequence substantially complementary
to or homologous with the sequence to be detected.
However, such base sequence need not be a single
continuous polynucleotide segment, but can be
comprised of two or more individual segments
interrupted by nonhomologous sequences. These
nonhomologous sequences can be linear, or they can
be self-complementary and form hairpin loops. In
addition, the homologous region of the probe can be
flanked at the 3'- and 5'-termini by nonhomologous
sequences, such as those comprising the DNA or RNA
of a vector into which the homologous sequence had
been inserted for propagation. In either instance,
the probe as presented as an analytical reagent
will exhibit detectable hybridization at one or
more points with sample nucleic acids of interest.
Linear or circular single stranded polynucleotides
can be used as the probe element, with major or
minor portions being duplexed with a complementary
polynucleotide strand or strands, provided that the
critical homologous segment or segments are in
single stranded form and available for
hybridization with sample DNA or RNA. It will
generally be preferred to employ probes which are
substantially in single stranded form. The
preparation of a suitable probe for a particular
assay is a matter of routine skill in the art.

MS-1362

*The Antibody Reagent*

A critical feature of the present invention is the ability to immobilize selectively hybrids formed under the favorable kinetics of interaction of the probe and sequences to be detected in solution. In contrast with prior art methods, the present invention uniquely provides for such immobilization by binding of formed hybrids to an antibody reagent which is added to the reaction medium in an immobilized state or which can be rendered immobilized in a subsequent step by conventional methods.

As used herein, antibody reagent refers to an immunologically derived binding substance having anti-hybrid binding activity and can be whole antibodies or fragments thereof, or aggregates or conjugates thereof, of the conventional polyclonal or monoclonal variety. Preferred antibody reagents will be those that are selective for binding double stranded nucleic acids over single stranded nucleic acids, e.g., those which selectively bind (i) DNA·RNA or RNA·RNA hybrids or (ii) intercalation complexes.

It is currently known that antibodies can be stimulated which are selective for DNA·RNA or RNA·RNA hybrids over the single stranded nucleic acids, however, it is presently considered infeasible to generate such selectivity in the case of DNA·DNA hybrids. To the extent that selective DNA·DNA antibodies are developed in the future, they will clearly be applicable to the present invention. Antibodies to DNA·RNA or RNA·RNA

MS-1362

hybrids can be used where the probe is RNA and the sample nucleic acids are DNA or RNA or where the probe is DNA and the sample RNA.

Further, it should be understood that in referring to an RNA probe used with an anti-DNA·RNA or anti-RNA·RNA reagent, it is contemplated herein that not all nucleotides comprised in the probe be ribonucleotides, i.e., bearing a 2'-hydroxyl group. The fundamental feature of an RNA probe as used herein is that it be sufficiently non-DNA in character to enable the stimulation of antibodies to DNA·RNA or RNA·RNA hybrids comprising an RNA probe which do not crossreact to an analytically significant degree with the individual single strands forming such hybrids. Therefore, one or more of the 2'-positions on the nucleotides comprised in the probe can be in the deoxy form provided the antibody binding characteristics necessary for performance of the present assay are maintained to a substantial degree. Likewise, in addition or alternatively to such limited 2'-deoxy modification, an RNA probe can comprise nucleotides having other 2'-modifications, or in general any other modification along its ribose phosphate backbone provided there is not substantial interference with the specificity of the antibody to the double stranded hybridization product compared to its individual single strands.

Where such modifications exist in an RNA probe, the immunogen used to raise the antibody reagent would preferably comprise one strand having substantially corresponding modifications and the other strand being substantially unmodified RNA or DNA, depending on whether sample RNA or DNA was

MS-1362

0146039

intended to be detected.  Preferably, the modified strand in the immunogen would be identical to the modified strand in an RNA probe.  An example of an immunogen is the hybrid poly(2'-0-methyladenylic acid)·poly(2'-deoxythymidylic acid).  Another would. be poly(2'-0-ethylinosinic acid)·poly(ribocytidylic acid).  The following are further examples of modified nucleotides which could be comprised in an RNA probe:  2'-0-methylribonucleotide, 2-0 -ethylribonucleotide, 2'-azidodeoxyribonucleotide, 2'-chlorodeoxyribonucleotide, 2'-0 -acetylribonucleotide, and the methylphosphonates or phosphorothiolates of ribonucleotides or deoxyribonucleotides.  Modified nucleotides can appear in RNA probes as a result of introduction during enzymic synthesis of the probe from a template.  For example, adenosine 5'-0-(1-thiotriphosphate) (ATPαS) and dATPαS are substrates for DNA dependent RNA polymerases and DNA polymerases, respectively.  Alternatively, the chemical modification can be introduced after the probe has been prepared.  For example, an RNA probe can be 2'-0-acetylated with acetic anhydride under mild conditions in an aqueous solvent.

Immunogens for stimulating antibodies specific for RNA·DNA hybrids can comprise homopolymeric or heteropolymeric polynucleotide duplexes.  Among the possible homopolymer duplexes, particularly preferred is poly(rA)·poly(dT) [Kitagawa and Stollar (1982) Mol. Immunol. 19:413].  However, in general, heteropolymer duplexes will be preferably used and can be prepared in a variety of ways, including transcription of ⌀X174 virion DNA with RNA polymerase [Nakazato (1980) Biochem. 19:2835].

MS-1362

The selected RNA·DNA duplexes are adsorbed to a methylated protein, or otherwise linked to a conventional immunogenic carrier material, such as bovine serum albumin, and injected into the desired host animal [see also Stollar (1980) Meth. Enzymol. 70:70]. Antibodies to RNA·RNA duplexes can be raised against double stranded RNAs from viruses such as reovirus or Fiji disease virus which infects sugar cane, among others. Also, homopolymer duplexes such as poly(rI)·poly(rC) or poly(rA)·poly(rU), among others, can be used for immunization as above. Further information regarding antibodies to RNA·DNA and RNA·RNA hybrids is provided in commonly assigned U.S. Patent Application Serial No. 616,132, filed June 1, 1984.

Antibodies to intercalation complexes can be prepared against an immunogen which will usually comprise an ionic complex between a cationic protein or protein derivative (e.g., methylated bovine serum albumin) and the anionic intercalator-nucleic acid complex. Ideally, the intercalator will be covalently coupled to the double stranded nucleic acid. Alternatively, the intercalator-nucleic acid conjugate can be covalently coupled to a carrier protein. The nucleic acid portion of the immunogen can comprise the specific paired sequences found in the assay hybrid or can comprise any other desirable sequences since the specificity of the antibody will generally not be dependent upon the particular base sequences involved. Further information regarding antibodies to intercalation complexes is provided in commonly assigned U.S. Patent

MS-1362

0146039

Application Serial No. 560,429, filed December 12, 1983.

As stated above, the antibody reagent can consist of whole antibodies, antibody fragments, polyfunctional antibody aggregates, or in general any substance comprising one or more specific binding sites from an antibody. When in the form of whole antibody, it can belong to any of the classes and subclasses of known immunoglobulins, e.g., IgG, IgM, and so forth. Any fragment of any such antibody which retains specific binding affinity for the hybridized probe can also be employed, for instance, the fragments of IgG conventionally known as Fab, $F(ab')$, and $F(ab')_2$. In addition, aggregates, polymers, derivatives and conjugates of immunoglobulins or their fragments can be used where appropriate.

The immunoglobulin source for the antibody reagent can be obtained in any available manner such as conventional antiserum and monoclonal techniques. Antiserum can be obtained by well-established techniques involving immunization of an animal, such as a mouse, rabbit, guinea pig or goat, with an appropriate immunogen. The immunoglobulins can also be obtained by somatic cell hybridization techniques, such resulting in what are commonly referred to as monoclonal antibodies, also involving the use of an appropriate immunogen.

In those instances where an antibody reagent selective for intercalation complexes is employed as the anti-hybrid, a variety of intercalator compounds can be involved. In general it can be said that the intercalator compound preferably is a

MS-1362

low molecular weight, planar, usually aromatic but sometimes polycyclic, molecule capable of binding with double stranded nucleic acids, e.g., DNA·DNA, DNA·RNA, or RNA·RNA duplexes, usually by insertion between base pairs. The primary binding mechanism will usually be noncovalent, with covalent binding occurring as a second step where the intercalator has reactive or activatable chemical groups which will form covalent bonds with neighboring chemical groups on one or both of the intercalated duplex strands. The result of intercalation is the spreading of adjacent base pairs to about twice their normal separation distance, leading to an increase in molecular length of the duplex. Further, unwinding of the double helix of about 12 to 36 degrees must occur in order to accomodate the intercalator. General reviews and further information can be obtained from Lerman, J. Mol. Biol. 3:18(1961); Bloomfield et al, "Physical Chemistry of Nucleic Acids", Chapter 7, pp. 429-476, Harper and Rowe, NY(1974); Waring, Nature 219:1320 (1968); Hartmann et al, Angew. Chem., Engl. Ed. 7:693(1968); Lippard, Accts. Chem. Res. 11:211(1978); Wilson, Intercalation Chemistry(1982),445; and Berman et al, Ann. Rev. Biophys. Bioeng. 20:87(1981); as well as from the above-referenced U.S. Serial No. 560,429. Exemplary of intercalators are acridine dyes, e.g., acridine orange, the phenanthridines, e.g., ethidium, the phenazines, furocoumarins, phenothiazines, and quinolines.

The intercalation complexes are formed in the assay medium during hybridization by use of a probe which has been modified in its complementary,

MS-1362

single stranded region to have the intercalator chemically linked thereto such that upon hybridization the intercalation complexes are formed. Essentially any convenient method can be used to accomplish such linkage. Usually, the linkage is formed by effecting intercalation with a reactive, preferably photoreactive intercalator, followed by the linking reaction. A particularly useful method involves the azidointercalators. Upon exposure to ultraviolet or visible light, the reactive nitrenes are readily generated. The nitrenes of arylazides prefer insertion reactions over their rearrangement products [see White et al, Methods in Enzymol. 46:644(1977)]. Representative azidointercalators are 3-azidoacridine, 9-azidoacridine, ethidium monoazide, ethidium diazide, ethidium dimer azide [Mitchell et al, JACS 104:4265(1982)], 4-azido-7-chloroquinoline, and 2-azidofluorene. Other useful photoreactable intercalators are the furocoumarins which form [2+2] cycloadducts with pyrimidine residues. Alkylating agents can also be used such as bischloroethylamines and epoxides or aziridines, e.g., aflatoxins, polycyclic hydrocarbon epoxides, mitomycin, and norphillin A. The intercalator-modified duplex is then denatured to yield the modified single stranded probe.

While as stated above preferred anti-hybrids for use in the present method will discriminate in their binding between double stranded and single stranded nucleic acids, it is not critical to have such selectivity for all embodiments. Where such selectivity is not critical, a variety of additional approaches for obtaining anti-hybrid become available. These include anti-DNA·DNA antibodies which are essentially nonspecific for DNA whether single or double stranded, antibodies raised against chemically modified nucleic acids such as cis-diaminedichloroplatinium-DNA adducts, antibodies to $OsO_4$ oxidized DNA such as anti-thymine glycol, antibodies to antibiotic-DNA complexes such as anti-distamycin A-DNA complex or anti-netropsin-DNA complex and antibodies to modified bases such as anti-5-bromouracil and anti-6-methyladenosine. The criticality of double stranded selectivity exists whenever the format of the assay involves the labeling of single stranded nucleic acid and thus requires discrimination between hybridized and unhybridized labeled nucleic acid. Such a situation is presented when either a probe or single stranded sample nucleic acids are labeled. On the other hand, when a labeled form of a second anti-hybrid reagent is employed, it is only necessary that one or the other of the ultimately immobilized anti-hybrid and the labeled anti-hybrid have selectivity for double stranded nucleic acids over single stranded nucleic acids.

While the present method has been particularly described as involving the immobilization of hybrids by the binding of antibody reagents, it will be recognized that essentially any substance which has the ability to selectively bind the nucleic acid hybrids formed during the assay may be likewise used. This applies both to those assay formats requiring selectivity for double stranded nucleic acids as well as where double versus single stranded selectivity is not critical as discussed above. Equivalents of the antibody reagent of the

MS-1362

present invention which can be used without departing from the present inventive concept will normally exhibit a highly specific noncovalent binding, as in ligand-receptor and immunoglobulin binding. Materials available now or in the future having such characteristic binding are contemplated as equivalents to the present antibody reagent.

The antibody reagent is brought into contact with the formed hybrids either in an immobilized form or an immobilizable form, i.e., can thereafter be rendered immobilized by conventional methods. It will generally be preferably to employ immobilized forms of the anti-hybrid. A large variety of methods are known for immobilizing proteins on solid supports and most of the methods are applicable to antibodies [see Methods in Enzymology, Vol. 44 (1976)]. Antibodies are commonly immobilized either by covalent coupling or by noncovalent adsorption. Noncovalent methods frequently employed are nonspecific adsorption to polystyrene beads or microparticles and to polyvinylchloride surfaces. Many covalent methods are used and a few include cyanogen bromide activated agaroses and dextrans; glutaraldehyde activated nylons and polyacrylamides; and epoxides on acrylic and other supports. Antibodies of the IgG class can also be immobilized by the binding to immobilized forms of protein A. Where protein A is used for this immobilization and labeled anti-hybrid will be used in the assay, care will be taken to prevent the labeled anti-hybrid from also being bound by the immobilized protein A such as by saturating the protein A binding sites or utilizing a labeled anti-hybrid which does not comprise the

MS-1362

protein A receptor site, e.g., by using Fab or Fab' or an anti-hybrid from an immunoglobulin class other than IgG.

The primary purpose of immobilization is to enable physical manipulation and isolation or separation of hybrids formed in the assay in order to measure them by the response or signal produced by the label. Thus, immobilization can occur subsequent to the binding of anti-hybrid to hybrids formed in the assay. A variety of ways to accomplish this are apparent.

One can employ immobilized anti-(anti-hybrid) antibodies, taking care that if labeled anti-hybrid is to be used in the assay that the immobilized antibodies do not have significant affinity for the labeled reagent. This can be accomplished by using antigenically distinguishable immunoglobulins for the two anti-hybrid reagents such as using anti-hybrids from different immunoglobulin classes. Immobilized protein A can also be employed with the same precautions as discussed above regarding the use of protein A and labeled anti-hybrid. Particularly useful are schemes involving the chemical modification of the anti-hybrid by conjugation to one member of a specific binding pair and the use of an immobilized form of the other member of the pair. Useful binding pairs from which to choose include biotin/avidin, haptens and antigens/antibodies, carbohydrates/lectins, enzymes/inhibitors, and the like. It is preferable to modify the anti-hybrid with biotin or a hapten and employ an immobilized form of avidin or anti-hapten antibody, respectively.

*Detection Labels*

The hybrids of interest that ultimately become associated with the immobilized antibody reagent can be determined in a variety of ways. It will generally be preferred to employ a labeled probe or a labeled form of a second antibody reagent capable of binding the formed hybrid. The latter is particularly preferred because neither the probe nor the sample nucleic acids need be chemically modified such as by being labeled in order to conduct the assay. One can simply contact the hybrids with the immobilized or immobilizable first antibody reagent and with the labeled second antibody reagent and measure the label that becomes associated with the immobilized phase or alternatively measure the label remaining as unbound labeled second antibody reagent. It is possible to use the same substance as the first and second antibody reagents. For instance, one can use anti-DNA·RNA where DNA·RNA hybrids are formed and one fraction of such anti-hybrids can be immobilized and another fraction labeled. The hybrids will have multiple epitopes for anti-DNA·RNA to allow for binding of both labeled and immobilized anti-hybrid. Alternatively, different anti-hybrids can be used, for example, where monoclonal antibodies are used one can employ different hybridomas for different binding specificities and/or affinities.

Labeled probes can be used in the present invention in several ways. In one case, a single labeled probe is used and the resulting labeled hybrids are separated from the free labeled probe for detection by binding to immobilized or immobilizable anti-hybrid. Alternatively, one can employ a dual hybridization approach involving two probe segments which are complementary to mutually exclusive segments on the sequence to be detected. One of the probe segments will be labeled and the other will form epitopes for anti-hybrid upon hybridization with the sequence to be detected.

A variety of other labeling schemes will be evident. For instance, although significantly less desirable one can introduce the label by labeling the single stranded sample nucleic acids *in situ* thus yielding labeled hybrids upon hybridization with the unlabeled probe. Also, as will be discussed in more detail below, the component to be labeled can be linked directly, e.g., by covalent bonds, to the substance that provides the ultimately detectable signal or by indirect linkages such as by incorporation of the ultimately detectable substance in a microcapsule or liposome which in turn is linked to the component to be labeled. Further, it will be understood that one can label with a specifically bindable ligand, e.g., a hapten or biotin, and bring in the ultimately detectable substance at any desirable time in the assay by addition of a binding partner for the ligand, e.g., an antibody or avidin, to which the detectable species is linked. When using immobilized and labeled anti-hybrids, they can be added to the reaction mixture simultaneously; however, depending on the particular system, performance might be optimized by adding one of the components a predetermined period of time before the other.

Labels of various types can be used to label probes, second anti-hybrid, and so forth as

MS-1362

described above. The label will be a substance which has a detectable physical, chemical, or electrical property. Such materials have been well-developed in the field of immunoassays and in general most any label useful in such methods can be applied to the present invention. Particularly useful are enzymatically active groups, such as enzymes (see Clin. Chem.(1976)22:1232, U.S. Reissue Pat. 31,006, and UK Pat. 2,019,408), enzyme substrates (see British Pat. Spec. 1,548,741), coenzymes (see U.S. Pat. Nos. 4,230,797 and 4,238,565), and enzyme inhibitors (see U.S. Pat. No. 4,134,792); fluorescers (see Clin. Chem.(1979)25:353); chromophores; luminescers such as chemiluminescers and bioluminescers (see Clin. Chem.(1979)25:512, and ibid, 1531); specifically bindable ligands such as biotin (see European Pat. Spec. 63,879) or a hapten (see PCT Publ. 83-2286); and radioisotopes such as $^3$H, $^{35}$S, $^{32}$P, $^{125}$I, and $^{14}$C. Such labels are detected on the basis of their own physical properties (e.g., fluorescers, chromophores and radioisotopes) or their reactive or binding properties (e.g., enzymes, substrates, coenzymes and inhibitors). For example, a cofactor-labeled species can be detected by adding the enzyme (or enzymes where a cycling system is used) for which the label is a cofactor and a substrate or substrates for the enzyme. A hapten or ligand (e.g., biotin) labeled species can be detected by adding an antibody to the hapten or a protein (e.g., avidin) which binds the ligand, tagged with a detectable molecule. Such detectable molecule can be some molecule with a measurable physical property (e.g., fluorescence or

MS-1362

absorbance) or a participant in an enzyme reaction
(e.g., see above list). For example, one can use
an enzyme which acts upon a substrate to generate a
product with a measurable physical property.
Examples of the latter include, but are not limited
to, β-galactosidase, alkaline phosphatase and
peroxidase.

Where a second antibody reagent is used to
measure the duplexes that become associated with
the immobilized first antibody reagent, such second
antibody can be detected based on a native property
such as its own antigenicity. A labeled
anti-(antibody) antibody will bind to the second
antibody reagent where the label for the second
antibody is any conventional label as above.
Further, antibody can be detected by complement
fixation or the use of labeled protein A, as well
as other techniques known in the art for detecting
antibodies. Where labeled protein A is used, the
immobilized antibody reagent will be in a form that
lacks the protein A binding site (e.g., one can use
immobilized Fab or Fab').

Methods for preparing the labeled components
used in the preferred embodiments of the present
invention are readily available from the prior art.
Most preferred are those assay formats which
involve labeled probes or labeled second
anti-hybrid. The labels described above can be
used for these embodiments. When labeling probes
one will employ synthetic approaches which are
effective for modifying nucleic acids without
substantially interfering with the ability of the
labeled probe to participate in hybridization, and
will select labels which are sufficiently stable

0146039

under the conditions to be used for hybridization
to enable their subsequent detection.

By way of example, the following approaches
can be used in labeling probes.  Radiolabeled
nucleotides can be incorporated into DNA probes by
methods such as nick translation, terminal labeling
with terminal deoxynucleotidyl transferase, and *in
vivo* labeling.  Radiolabeled nucleotides can be
incorporated into RNA probes during *in vitro*
synthesis with DNA dependent RNA polymerase from
bacteriophage SP6 using the Riboprobe[TM] DNA
template system from Promega Biotec, Madison, WI.
The method of Langer et al [(1981) Proc. Nat'l.
Acad. Sci., 78:6633] can be used to couple biotin
to the primary amine of 5-(3-amino)allyluridine and
deoxyuridine triphosphates.  These biotinylated
nucleotides can be incorporated into double
stranded DNA by nick translation or added to the
3'-OH terminus with terminal deoxynucleotidyl
transferase.  Biotin can also be attached to the
3'-OH terminus of RNA through polyamine [Broker,
T.R., (1978) Nucl. Acids Res. 4:363] and cytochrome
C bridges [Sodja, A. and Davidson, N. (1978) Nucl.
Acids. Res. 5:385].  Direct coupling of protein
labels to probes can be accomplished by the method
of Renz [(1983) EMBO Journal, 2:817] who coupled
$^{125}$I-histones to denatured DNA with glutaraldehyde.
Enzymes such as peroxidase and alkaline phosphatase
can be linked to DNA probes by means of similar
chemistry [Renz and Kurz (1984) Nucl. Acids Res.
12:3435].  Other chemistries for end-labeling DNA
probes include that described by Eshaghpour et al
[(1979) Nucl. Acids Res. 7:1485].  One or more
4-thiouridine residues can be introduced on the 3'-OH ends of DNA and the thiols reacted with various electrophilic low molecular weight reagents. This chemistry can be used to attach various haptens to DNA probes. Labeling with the hapten N-acetoxy-N-2-acetylaminofluorene is described by Tchen et al [(1984) Proc. Nat'l. Acad. Sci. 81:3466]. DNA and RNA probes can be reacted with N-acetoxy-N-2-acetylaminofluorene to yield an adduct having N-2-acetylaminofluorene residues attached at the 8-carbon of guanine. The covalently modified DNA can be detected with antibody raised against the N-acetoxy-N-2-acetyl-aminofluorene residue. The method of Hu and Messing [(1982) Gene, 17:271] can be used for adding labels to probes cloned into single stranded M13 vectors. A universal primer, complementary to the region 5' to the cloning site, initiates DNA synthesis complementary to the M13 strand downstream from the probe sequence. Since the DNA polymerase will incorporate radioactive nucleotide triphosphates and biotin 5-(3-aminoallyl) deoxyuridine triphosphate into the new strand, those labels can be attached to the vector away from the probe sequence. The double stranded portion can also be modified by reaction with 8-azidoethidium.

The preparation of labeled antibodies is described extensively in the literature. Incorporation of $^{125}$I-label can be accomplished by the method of Balton and Hunter (1973) Biochem. J. 133:529. Ishikawa et al (1983) J. Immunoassay 4:209 have outlined several different methods for coupling various enzymes to antibodies. Yoshitake et al (1979) Eur. J. Biochem. 101:395 have

MS-1362

described a method for using maleimides to couple glucose oxidase to antibody. Alkaline phosphatase can be coupled to antibody with glutaraldehyde [Voller et al (1976) Bull. World Health Organ., 53:55]. Antibodies can be labeled with fluorescein by the method of Blakeslee and Baines (1976) J. Immunol. Meth., 13:305. Chemiluminescent labels can be introduced by the method of Schroeder et al (1981) Clin. Chem. 27:1378.

With reference to the drawings and the examples which follow, a few specific embodiments of the present assay can be described.

The method illustrated in Fig. 1 involves the use of an unlabeled polynucleotide probe which is either RNA or DNA when the sample sequence of interest is RNA or is RNA when the sample sequence is DNA. Two populations of anti-hybrid antibodies (Ab) are employed which can comprise the same or different polyclonal or monoclonal immunoglobulins selective for RNA·RNA or RNA·DNA hybrids, as the case may be. The first anti-hybrid reagent is presented in an immobilized form and the second is labeled with a fluorescent substance. Upon formation of hybrids between the sequence of interest and the probe, binding sites for the anti-hybrid reagents are formed. As a result, the hybrids become immobilized through binding of the immobilized anti-hybrid and fluorescently labeled through binding of the labeled anti-hybrid. The resulting immobilized and labeled hybrids are separated from unbound labeled anti-hybrid and the amount of fluorescence produced by the immobile species is measured and correlated with the presence or amount of the sequence of interest in the sample.

In the method shown in Fig. 2, the probe is doubly modified, both with a biotin label (bio) and a chemically linked intercalator (I). An antibody (Ab) to intercalation complexes serves as the critical anti-hybrid reagent and is presented in an immobilized form. The biotin-label is detected by use of avidin (Av) labeled with a detectable enzyme. Upon completion of the hybridization step, the resulting immobilized and labeled hybrids are separated from unbound labeled avidin and the enzyme activity of the immobile species is measured and related to the presence of the sequence of interest.

*Reaction Mixture*

The test sample to be assayed can be any medium of interest, and will usually be a liquid sample of medical, veterinary, environmental, nutritional, or industrial significance. Human and animal specimens and body fluids particularly can be assayed by the present method, including urine, blood (serum or plasma), milk, cerebrospinal fluid, sputum, fecal matter, lung aspirates, throat swabs, genital swabs and exudates, rectal swabs, and nasopharnygal aspirates. Where the test sample obtained from the patient or other source to be tested contains principally double stranded nucleic acids, such as contained in cells, the sample will be treated to denature the nucleic acids, and if necessary first to release nucleic acids from cells. Denaturation of nucleic acids is preferably

MS-1362

accomplished by heating in boiling water or alkali treatment (e.g., 0.1 N sodium hydroxide), which if desired, can simultaneously be used to lyse cells. Also, release of nucleic acids can, for example, be obtained by mechanical disruption (freeze/thaw, abrasion, sonication), physical/chemical disruption (detergents such as Triton, Tween, sodium dodecylsulfate, alkali treatment osmotic shock, or heat), or enzymatic lysis (lysozyme, proteinase K, pepsin). The resulting test medium will contain nucleic acids in single stranded form which can then be assayed according to the present hybridization method. In those situations where RNA·DNA hybrids are to be detected with labeled antibody reagents, mRNA and rRNA in the sample can be removed from participating in the hybridization reactions by conventional methods such as treatment with alkaline conditions, e.g., the same conditions used to denature the nucleic acids in the sample.

As is known in the art, various hybridization conditions can be employed in the assay. Typically, hybridization will proceed at slightly elevated temperatures, e.g., between about 35 and 75°C and usually around 65°C, in a solution comprising buffer at pH between about 6 and 8 and with appropriate ionic strength (e.g., 5XSSC where 1XSSC = 0.15M sodium chloride and 0.015M sodium citrate, pH 7.0) and optionally protein such as bovine serum albumin, and a denatured foreign DNA such as from calf thymus or salmon sperm. In cases where lower hybridization temperatures are desirable, hydrogen bonding reagents such as dimethyl sulfoxide and formamide can be included. The degree of complementarity between the sample and probe strands required for hybridization to occur depends on the stringency of the conditions. Factors which determine stringency are known in the art.

Normally, the temperature conditions selected for hybridization will be incompatible with the binding of the anti-hybrid reagent to formed hyrids and detection of the label response. Accordingly, the anti-hybrid binding step and label detection step will proceed after completion of the hybridization step. The reaction mixture will usually be brought to a temperature in the range of from about 3°C to about 40°C and the binding and detection steps then performed. Dilution of the hybridization mixture prior to addition of anti-hybrids is desirable when the salt and/or formamide concentrations are high enough to interfere significantly with the antibody binding reaction.

*Reagent System*

The present invention additionally provides a reagent system, i.e., reagent combination or means, comprising all of the essential elements required to conduct a desired assay method. The reagent system is presented in a commercially packaged form, as a composition or admixture where the compatability of the reagents will allow, in a test device configuration, or more usually as a test kit, i.e., a packaged combination of one or more containers, devices, or the like holding the necessary reagents, and usually including written instructions for the performance of assays.

MS-1362

Reagent systems of the present invention include all configurations and compositions for performing the various hybridization formats described herein.

In all cases, the reagent system will comprise (1) a nucleic acid probe as described herein, and (2) the anti-hybrid reagent. A test kit form of the system can additionally include ancillary chemicals such as the components of the hybridization solution and denaturation agents capable of converting double stranded nucleic acids in a test sample into single stranded form. Preferably, there is included a chemical lysing and denaturing agent, e.g., alkali, for treating the sample to release single stranded nucleic acid therefrom.

The present invention will now be illustrated, but is not intended to be limited, by the following examples.

MS-1362

*Example I*

Hybridization Assay Using Immobilized
and Labeled Anti-Hybrids

A.   Preparation of an RNA probe for cytomegalovirus

A DNA fragment from the genome of cylomegalovirus is cloned into a vector containing the promoter for DNA dependent RNA polymerase from bacteriophage SP6 that infects *Salmonella typhimurium* LT2 cells.   The cloned sequence is transcribed by the polymerase to produce an RNA probe.

Cytomegalovirus DNA is digested with EcoRI restriction endonuclease and the fragments are cloned into the plasmid pACYC 184 [Tamashiro et al (1982) J. Virology 42:547-557].   The plasmids are propagated in E. coli strain HB101 and the EcoRI e fragment defined in the Tamashiro reference is used for preparation of the probe.   The purified plasmid is digested with EcoRI restriction endonuclease and the insert is isolated by agarose gel electrophoresis [Maniatis et al (1982) "Molecular Cloning", Cold Spring Harbor Laboratory].   The cytomegalovirus EcoRI e fragment is cloned into the EcoRI site of the pSP65 vector available from Promega Biotec, Madison, WI.

The pSP65 vector with the EcoRI e insert is propagated in E. coli JM103 cells at 37°C.   The cells are lysed and the cellular DNA is isolated by phenol/chloroform extractions.   The plasmid is separated from the chromosomal DNA by centrifugation in a cesium chloride-ethidium
bromide gradient [Maniatis et al, *supra*].

The cesium chloride and ethidium bromide are
separated from the plasmid by gel filtration on
Sephadex G-50 (Pharmacia Fine Chemicals,
Piscataway, NJ) in 10 mM Tris-hydrochloride buffer,
pH 7.5, containing 50 mM NaCl and 1 mM EDTA.  The
effluent containing DNA is collected and the DNA is
precipitated with cold ethanol.  The precipitate is
dissolved in 10 mM Tris-hydrochloride buffer, pH
7.5, containing 50 mM NaCl, 10 mM $MgCl_2$ and 1 mM
dithiothreitol and digested for 1 hour at 37°C with
1 unit Hind III restriction endonuclease per
microgram DNA.  The mixture is extracted once with
phenol/chloroform and the DNA is precipitated with
cold ethanol.  The precipitate is dissolved in 10
mM Tris-hydrochloride buffer, pH 7.4, to give 0.5
mg DNA/mL.

This pSP65 vector with the EcoRI e insert is
transcribed with SP6 DNA dependent RNA polymerase
starting at the promoter and ending at the site cut
by Hind III restriction endonuclease.  Most of the
DNA in the transcribed region is the EcoRI e
insert.

The transcription reaction mixture (500 µL)
has the following compositions:  50 µg of the Hind
III digested DNA; 40 mM Tris-hydrochloride buffer,
pH 7.5; 6 mM $MgCl_2$; 2 mM spermine; 0.5 mM ATP, CTP,
UTP and GTP; 10 mM dithiothreitol; 500 units RNasin
(Promega Biotec) and 50 units of SP6 RNA polymerase
(Promega Biotec).  The reaction is allowed to
proceed for 1 hour at room temperature and then an
additional 50 units of RNA polymerase is added and
reacted for one hour.

MS-1362

DNA in the mixture is destroyed by digestion for 10 minutes at 37°C with 10 µg of RNase-free DNase. The reaction mixture is extracted with phenol/chloroform and chromatographed on Sephadex G-50 in 10 mM Tris-hydrochloride buffer, pH 7.4, 0.1 M NaCl. The RNA is collected and precipitated with cold ethanol. The precipitate is dissolved in 50 mM sodium acetate buffer, pH 6.0, containing 1 mM EDTA.

B.    Preparation of monoclonal antibody to RNA·DNA

1.    Preparation of RNA·DNA hybrid - The hybrid is prepared by transcription of $\phi$X174 virion DNA with DNA dependent RNA polymerase from E. coli. The procedure is described by Nakazato (1980) Biochem 19:2835.

2.    Preparation of methylated thyroglobulin - Bovine thyroglobulin (Sigma Chemical Co., St. Louis, MO), 100 mg, is combined with 10 ml of anhydrous methanol and 400 µL of 2.5 M HCl in methanol. The mixture is allowed to react for 5 days on a rotary mixer at room temperature. The precipitate is collected by centrifugation and washed twice with methanol and twice with ethanol. Then it is dried under vacuum overnight.

3.    Immunization of mice - One hundred fifty micrograms of RNA·DNA hybrid in 250 µL of 20 mM Tris-hydrochloride buffer, pH 7.4, 1 mM EDTA is combined with 150 µg of methylated thyroglobulin in 250 µL water. A precipitate forms and 2.5 ml of the Tris buffer is added. The entire mixture is

MS-1362

emulsified with an equal volume of Freunds adjuvant. BALB/c mice are each immunized with 0.5 ml of the suspension. Booster immunizations are given 3 weeks later and at one week intervals thereafter. Blood is taken at two week intervals beginning one week after the first boost.

Antibody titers in the serums are measured by an enzyme labeled immunosorbent assay method. Immulon II (Dynateck, Alexandria, VA) microtiter wells are coated with RNA·DNA by placing 50 $\mu$L of a 5 $\mu$g/ml solution in each well. The RNA·DNA is in 0.015 M sodium citrate buffer, pH 6.8, containing 0.15 M NaCl. When the solutions have stood at room temperature for 2 hours, the wells are washed with 0.02 M sodium phosphate buffer, pH 7.4, containing 5 mg bovine serum albumin/mL and 0.5% Tween 20 detergent (v/v). Appropriate dilutions of antiserums are added to the wells to allow binding of antibodies to the immobilized RNA·DNA. Then the bound antibodies are detected with enzyme labeled anti-mouse IgG by well-known procedures. Spleen cells from a mouse with a high serum titer to RNA·DNA but low titer to single stranded DNA are fused with myeloma cells to produce hybridomas [Stuart et al (1981) Proc. Natl. Acad. Sci. USA. 78:3751; Galfre and Milstein (1981) Meth. in Enzymol. 73:1].

Cloned hybridomas are grown intraperitoneally in mice to produce adequate quantities of antibody for further work. Albumin is removed from the ascites fluid by chromatography on Affigel-Blue® resin (Bio-Rad Laboratories, Richmond, CA) equilibrated with 10 mM Tris-hydrochloride buffer, pH 8.0, 0.15 M NaCl. The column effluent

MS-1362

containing antibody is chromatographed on DEAE-Sepharose (Pharmacia Fine Chemicals, Piscataway, NJ) using a linear gradient from 10 mM Tris-hydrochloride buffer, pH 8.0, to this buffer containing 0.2 M NaCl. The major peak of eluted protein contains the monoclonal antibody free of transferrin and albumin.

C.    Immobilization of monoclonal antibody to RNA·DNA.

The monoclonal antibody to RNA·DNA is immobilized on magnetizable microparticles, Act -Magnogel AcA44, available from LKB Instruments, Gaithersburg, MD. The particles are porous polyacrylamide-agarose beads containing 7% iron oxide and this resin is activated with glutaraldehyde. Coupling of the antibody to Act-Magnogel AcA44 is carried out according to the manufacturer's instructions.

D.    Fluorescein labeled antibody to RNA·DNA

Purified antibody to RNA·DNA is dialyzed into 0.1 M sodium borate buffer, pH 9.0, and 0.5 ml containing 5 mg antibody is combined with 0.5 ml of 5-(4,6-dichlorotriazin-2-yl)-aminofluorescein (Molecular Probes, Inc., Junction City, OR) in the borate buffer. The mixture is allowed to react for 1 hour at 25°C and then chromatographed on a 1 x 25 cm Biogel P-6DG (Bio-Rad Laboratories) column with 0.1 M Tris-hydrochloride buffer, pH 8.0, as eluent. Absorbances at 280 nm of 1.0 ml fractions are monitored and those comprising the first peak are pooled. The fluorescein/protein ratio is determined by the method of The and Feltkamp (1970) Immunology 18:865.

#### E.  Hybridization assay for cytomegalovirus in urine

This assay involves the separation of cytomegalovirus from urine by centrifugation and hybridization of viral DNA with the RNA probe in solution. The amount of hybrid formed is determined immunochemically with the immobilized antibody to RNA·DNA and the fluorescein labeled antibody.

Cells and cellular debris are removed from urine by centrifugation in a Sorvall GLC-3 centrifuge at 3000 rpm for five minutes. Ten milliliters of supernatant is placed in a polyallomer ultracentrifuge tube and run at 25,000 rpm in a Beckman Ti50 rotor for 75 minutes. The supernatant is removed and the pellet is dissolved in 0.05 mL of 0.1 M NaOH and incubated at 37°C for 30 minutes.

One hundred fifty microliters of the following solution is added:  0.1 M sodium phosphate buffer, pH 6.0; 1.8 M NaCl; 0.1% sodium dodecylsulfate (w/v) and 1 mM EDTA. Then 20 µL of the RNA probe (20 ng) is added and the mixture is incubated at 65°C for 10 hours. The reaction is cooled to room temperature and 650 µL of 0.1 M sodium phosphate buffer, pH 7.4, containing 2 mM $MgCl_2$ and 5.0 mg bovine serum albumin/ml is added. Then 50 µL of the immobilized antibody is added and the mixture is agitated for 30 minutes in such a way as to keep the solid-phase antibody in suspension. Fifty

MS-1362

microliters of fluorescein labeled antibody is added and the agitation is continued for 1 hour. The concentrations of the immobilized antibody and the fluorescein labeled antibody reagents are optimized in preliminary experiments to provide antibody levels in large excess over those required to bind all of the RNA·DNA hybrid present in the assays.

At the end of the reaction period the solid support is attracted to one wall of the reaction tube with a magnet and the liquid is removed by aspiration. The solid phase is washed twice, 1 mL each, with 0.1 M sodium phosphate buffer, pH 7.4 containing 2.0 mM $MgCl_2$ and 0.1% Tween 20 (v/v).

The fluorescein labeled antibody bound to the solid phase is dissociated by addition of 1.0 mL of 0.1 M NaOH. The suspension is agitated for 5 minutes and the solid phase is attracted to the bottom of the tube with a magnet. The fluorescence of the solution is measured with 495 nm light for excitation and 520 nm for emission.

A control assay is run in parallel with a urine sample which is known to be free of cytomegalovirus. Fluorescence signals generated with the infected urine specimens will be higher than those for the controls.

*Example II*

## Hybridization Assay Using Immobilized
## Anti-Hybrid and Labeled Probe

A.    Preparation of a labeled probe for
      cytomegolovirus DNA

The EcoRI e fragment from cytomegolovirus described in Example I above is cloned into the M13mp8 vector available from New England Biolabs, Beverly, MA.  The recombinant virus is propagated in the E. coli K12JM101 host.  The virus is separated from the culture fluid by precipitation with polyethyleneglycol and the single stranded virion DNA is isolated by phenol/chloroform extraction.

A 17 base oligodeoxynucleotide primer with the sequence GTAAAACGACGGCCAGT is complementary to a segment of M13mp8 close to the 3'-OH end of the EcoRI e insert.  This primer will initiate DNA synthesis by the Klenow fragment of DNA polymerase I from E. coli and the replication is directed through the EcoRI e insert.  The new DNA probe is labeled with biotin by including Bio-11-dUTP (available from Enzo Biochem, Inc., New York, NY) in the reaction mixture in place of the usual dTTP [Leary et al (1983) Proc. Natl. Acad. Sci. 80:4045].

The purified M13mp8 DNA with the EcoRI e insert is combined with a molar excess of the 17 base primer (New England Biolabs) in 20 mM Tris-HCl buffer, pH 8.0, containing 10 mM $MgCl_2$ (final concentrations) [Bankier and Barrell, Techniques in

MS-1362

Nucleic Acid Biochemistry, Elsevier, Ireland]. The mixture is incubated at 55°C for 45 minutes to anneal the primer to the M13mp8 DNA. The reaction mixture is made 15 mM in dATP, dGTP, dCTP and Bio-11-dUTP and finally the Klenow fragment of DNA polymerase I is added. This reaction is incubated at 25°C for a period determined for each set of reagents. The reaction time is optimized to give newly synthesized DNA fragments somewhat larger than the EcoRI e insert. To determine the optimum time, samples are taken from the reaction mixture at various times and electrophoresed in denaturing alkaline agarose gel. [Maniatis et al, *supra*]. This procedure will provide biotin labeled DNAs with some variations in length; however, extension of the labeled DNA beyond the EcoRI e insert into the M13 sequence is acceptable for the present purpose.

The DNA is purified by phenol/chloroform extraction and precipitated with ethanol. It is dissolved in 20 mM Tris-hydrochloride buffer, pH 8.0, and ethidium residues are introduced as covalent intercalation complexes. For this purpose 8-azidoethidium is prepared and purified as described by Graves et al (1977) Biochim. Biophys. Acta 479:98. (Our studies show that this procedure gives a mixture of 3- and 8-azidoethidium isomers.)

A solution of the biotin labeled DNA complexed to the M13 template (about 50 µg DNA/mL) is made 0.5 mM in 8-azidoethidium and photolyzed for 1 hour at a distance 10 to 20 cm from a 150 watt outdoor spotlight. The DNA solution is stirred during the photolysis in a glass reaction tube which is in a glass water bath. The glass absorbs ultraviolet

MS-1362

- 41 -

0146039

radiation and the water bath is used to keep the reaction temperature between 20 and 30°C.

At the end of the photolysis the noncovalently bound ethidium photolysis products are removed by 10 successive extractions with water saturated n-butanol. Then the DNA is precipitated with ethanol and dissolved in the Tris buffer. The amount of covalently bound DNA is estimated by means of optical absorption measurements and the extinction coefficients of $E_{490} \simeq 4 \times 10^3$ $M^{-1}$ $CM^{-1}$ for photolyzed ethidium azide, the relationship between $A_{260}$ and $A_{490}$ for photolyzed ethidium bound to DNA $[A_{266} = (A_{490} \times 2.3) - 0.011]$ and $E_{260} \simeq 1.3 \times 10^4$ $M^{-1}$ $cm^{-1}$ for the DNA base pair concentration.

The ethidium will be covalently bound to the DNA almost exclusively in the double stranded region where intercalation complexes can form. The goal is to introduce one ethidium residue per 10 to 50 base pairs. The photolysis reaction goes nearly to completion during the one hour reaction period and the incorporation of ethidium can be decreased by reducing the photolysis time or reducing the ethidium concentration. Incorporation can be increased by repeating the photolysis with fresh 8-azidoethidium.

The final step is the separation of the biotin labeled and intercalator-modified probe from the M13 mp8 template DNA. The separation is accomplished by electrophoresis in alkaline agarose gel [Maniatis et al, *supra*]. The DNA bands are detected in the gel by fluorescent staining with ethidium bromide. The biotin labeled DNA is smaller than the M13 mp8 template strand and therefore migrates faster. Gel containing the biotin labeled DNA is excised and the DNA is recovered by electroelution as described in the Maniatis reference.

    B.    Preparation of monoclonal antibody to ethidium intercalated DNA

    1.    Preparation of covalent ethidium-DNA complexes

About 250 mg of salmon sperm DNA (Sigma Chemical Co., St. Louis, MO) is dissolved in 40 ml of 50 mM NaCl and sheared by five passages through a 23 gauge needle. The sheared DNA is placed in a 250 mL flask and diluted with an additional 160 mL of buffer. One hundred forty-five microliters (145 $\mu$L) of $S_1$-nuclease, 200,000 units per mL (Pharmacia P-L Biochemicals, Piscataway, NJ), is added and the mixture is incubated at 37°C for 50 minutes.

Then the reaction mixture is extracted twice with phenol:chloroform, once with chloroform and the DNA is precipitated twice with ethanol [Maniatis et al, *supra*]. The final precipitate is dissolved in 70 mL of 20 mM Tris hydrochloride buffer, pH 8.0.

This DNA is reacted with 8-azidoethidium under the following conditions. The reaction mixture is prepared with 33 ml of 2.7 mg DNA/mL, 13.5 mL of 4.95 mM 8-azidoethidium, 13.5 mL of 0.2 M Tris-hydrochloride buffer, pH 8.0, 0.2 M NaCl, and 76 mL water. The mixture is placed in a 250 mL beaker with a water jacket maintained at 22°C. The mixture is stirred and illuminated for 60 minutes by a 150 watt spotlight at a distance of 10 cm.

This photolysis is repeated with an identical reaction mixture.

The photolyzed reaction mixtures are combined and extracted 10-times with an equal volume each time of n-butanol saturated with 20 mM Tris-hydrochloride buffer, pH 8.0, 0.2 M NaCl. The extracted DNA solution is combined with 23 mL of 4.95 mM 8-azidoethidium and 77 mL of 20 mM Tris-hydrochloride buffer, pH 8.0, 0.2 M NaCl. This solution is photolyzed for 60 minutes as described above. The reaction products are extracted 10 times with buffer saturated butanol as described above and the DNA is precipitated with ethanol. The precipitate is dissolved in 10 mM Tris-hydrochloride buffer, pH 8.0, 1 mM EDTA and the absorbances at 260 and 490 nm are recorded.

2. Preparation of covalent ethidium-DNA methylated thyroglobulin complex.

Methylated thyroglobulin (5 mg) prepared as described in Example I, part B-2 above is dissolved in 1.0 mL water and 5.6 ml of a 2.2 mg/mL covalent ethidium DNA solution is added. A precipitate forms immediately and the suspension is diluted to 62.5 mL with 0.15 M NaCl. This suspension is emulsified with an equal volume of Freunds adjuvant and each BALB/c mouse is immunized subcutaneously with 0.5 mL of the mixture. Booster immunizations are given at 2 week intervals with the same mixture. Test bleedings are taken one week after each immunization beginning after the third boost. Antibody titers are assayed by standard enzyme label immunoadsorbant procedures. Covalently intercalated DNA, double stranded DNA and single stranded DNA are coated on microtiter wells as described in Example I, part B-3 above. Diluted antiserums are incubated in the coated wells and antibody bound to the immobilized antigens are detected as outlined above. Antiserums are screened for those which have low titers to single and double stranded DNA but high titers to covalently intercalated DNA.

In an additional experiment, antibody titers to noncovalent ethidium-DNA intercalation complex are measured. For this purpose, double stranded DNA is coated on the wells and 100 $\mu$M ethidium bromide is included in the diluted antiserum and all subsequent binding and wash solutions, but it is not included in the substrate solution used for measurement of enzyme activity.

Antiserums typically give higher titers to covalently intercalated DNA than to the noncovalent complex. This difference is due to the presence of antibodies to the ethidium residue which becomes covalently bound in low yield to the phosphate-ribose structure of double stranded DNA. Therefore animals with high titers to noncovalently intercalated DNA are chosen for preparation of hybridomas.

Spleen cells from the selected mice are fused with myeloma cells to produce hybridomas [Stuart et al, *supra*; Gilfre and Milstein, *supra*]. The hybridoma cells are cloned in selective medium and those which produce antibody to noncovalently intercalated DNA are selected for antibody production intraperitoneally in mice.

The antibody is isolated from the ascites fluid as described in Example I, part B-3 above.

C.    Preparation of β-Galactosidase-Streptavidin Conjugate

Sulfhydryl residues on β-galactosidase are exposed by reduction with dithiothreitol. β-Galactosidase (30,000 units, grade VIII, Sigma Chemical Co.) in 2 mL of 0.1 M N-2-hydroxyethyl-piperazine-N'-2-ethane sulfonate buffer (HEPES), pH 7.0, 0.09 M NaCl, is combined with 3.5 μmol of dithiothreitol and allowed to stand at room temperature for 4 hours. The dithiothreitol is removed by chromatography on a 2.5 x 80 cm column of Sepharose 6B Cl (Pharmacia Fine Chemicals) in the buffer described above. Fractions containing protein are combined into a pool. The number of moles of sulfhydryl groups per mole of enzyme are measured by the method of Ellman (1959) Arch. Biochem. Biophys. 82:70.

Succinimidyl-4-(N-maleimidomethyl)cyclohexane-1-carboxylate (SMCC) (Pierce Chemical Co., Rockford, IL), 5.3 mg, is dissolved in 250 μL of anhydrous N,N-dimethylformamide and a 40 μL aliquot is combined with 3 mL of 0.1 M HEPES buffer, pH 7.0, 0.15 M NaCl. A 25 μL aliquot of this aqueous solution is added to 825 μL of HEPES/NaCl buffer and 100 μL of 1 mM glutathione (reduced form). When this reaction mixture has stood at room temperature for 15 minutes, the unreacted glutathione is determined by Ellman's method. The results are used to calculate the SMCC concentration.

MS-1362

Streptavidin obtained from Bethseda Research Laboratories, Gaithersburg, MD, is exchanged into 0.1 M HEPES buffer, pH 7.0, 0.15 M NaCl by gel exclusion chromatography in Biogel P-6DG. Following exchange, 1.75 ml of 3.7 mg/mL streptavidin is combined with 17.6 µL of 61 mM SMCC and allowed to react for one hour at 30°C. The reaction mixture is chromatographed on a 1 x 25 cm column of Biogel P-6DG in the HEPES buffer. The fractions corresponding to the first effluent peak with absorbance at 280 nm are pooled and assayed for maleimide content by back titration of glutathione as outlined above.

Since the maleimide is subject to hydrolysis, coupling to β-galactosidase is initiated as soon as possible. Activated streptavidin, 3.9 mg, in 3.3 ml of the HEPES buffer is added to 32 mg of reduced β-galactosidase to give a reaction volume of 9.3 ml. After 4 hours at 25°C the reaction is quenched by adding 800 µL of 1 mM glutathione and incubated an additional 30 minutes at 25°C. Then the reaction mixture is chromatographed on a 1.5 x 110 cm column of Biogel A-1.5 m (Bio-Rad Laboratories) and developed with 0.1 M HEPES buffer, pH 7.0, 0.15 M NaCl. Two milliliter fractions are collected and absorbances at 280 nm are recorded. The first peak is pooled for further study.

D.     Immobilization of antibody to ethidium intercalated DNA.

The monoclonal antibody to ethidium intercalated DNA is immobilized on Act-Magnogel AcA44 as described in Example 1, part C above.

E.     Hybridization assay for cytomegalovirus in urine.

Processing of urine specimens and hybridization reactions are carried out as described in Example I, part E above except that the biotin labeled DNA probe (section A) is used in place of the RNA probe. Following the hybridization reaction, 650 µL of 0.1 M sodium phosphate buffer, pH 7.4, containing 2 mM $MgCl_2$, 5.0 mg bovine serum albumin/ml and the immobilized antibody to ethidium intercalated DNA (Part D above) is added. The mixture is agitated for 30 minutes in such a way as to keep the solid-phase antibody in suspension. Then 50 µL of β-galactosidase labeled streptavidin is added and the agitation is continued for one hour.

When the incubation is completed, the solid phase is attracted to one wall of the reaction tube with a magnet and the liquid is removed by aspiration. The solid phase is washed twice, 1 mL each, with 0.1 M sodium phosphate buffer, pH 7.4, containing 2.0 mM $MgCl_2$ and 0.1% Tween 20 (v/v).

The enzyme activity associated with the solid phase is measured by addition of 1.0 ml of 0.1 M sodium phosphate buffer, pH 7.4, containing 800 µM

MS-1362

7-β-galactosyl-3-[6-aminohexylcarboxamide] coumarin [Worah et al (1981) Clin. Chem. 27:673]. At the end of this incubation the solid phase particles are attracted to the bottom of the cuvette with a magnet and the fluorescence of the solution is recorded using 400 nanometers (nm) excitation and 450 nm emission.

A control assay is run in parallel with a urine sample which is known to be free of cytomegalovirus. The fluorescence signals generated with the infected urine specimens will be higher than the controls.

PROPOSED CLAIMS FOR EPO, DENMARK,
FINLAND, IRELAND, AND NORWAY

1. A method for detecting a particular polynucleotide sequence in a test sample comprising single stranded nucleic acids, comprising the steps of contacting the test sample with a nucleic acid probe comprising at least one single stranded base sequence that is substantially complementary to the sequence to be detected, under conditions favorable to hybridization between the probe and the sequence to be detected, and determining the resulting duplexes,

characterized in that the duplexes formed by hybridization between the complementary probe sequence and the sequence to be detected are contacted with an antibody reagent which is selective for binding such duplexes, the antibody reagent being immobilized or thereafter is rendered immobilized, and said duplexes that become bound to the immobilized antibody reagent are determined.

2. The method of Claim 1 wherein said antibody reagent is selective for binding (i) DNA·RNA hybrids wherein one of the probe and the sequence to be detected is DNA and the other is RNA, (ii) RNA·RNA hybrids wherein both the probe and the sequence to be detected are RNA, or (iii) intercalation complexes wherein the duplexes formed in the assaycomprise a nucleic acid intercalator bound thereto in the form of intercalation complexes.

3. The method of Claim 1 wherein the duplexes that become bound to the immobilized antibody reagent are determined by addition of a labeled form of a second antibody reagent capable of binding said duplexes, wherein the first and second antibody reagents are preferably the same substance, and the label that becomes associated with the immobilized reagent is measured.

MS-1362

4.    The method of Claim 1 wherein the probe is labeled and the duplexes that become bound to the immobilized antibody reagent are determined by measuring the label that has become associated with the immobilized reagent.

5.    The method of any one of Claims 1-4 wherein said antibody reagent is added in a soluble form and is linked to a specifically bindable ligand and wherein said resulting duplexes are additionally contacted with an immobilized form of a binding partner for said ligand.

6.    The method of Claim 5 wherein said ligand is biotin or a hapten and said binding partner is avidin or an anti-hapten antibody, respectively.

7.    The method of any one of Claims 1-4 wherein the label is an enzymatically active group, a fluorescer, a chromophore, a luminescer, a specifically bindable ligand, or a radioisotope.

8.    The method of Claim 1 wherein the test sample is a biological sample that has been subjected to conditions to release and denature nucleic acids present therein.

9.    A reagent system for detecting a particular polynucleotide sequence in a test sample, comprising nucleic acid probe comprising at least one single stranded base sequence that is substantially complementary to the sequence to be detected,
characterized by comprising additionally an antibody reagent which is selective for binding duplexes formed by hybridization between the complementary probe sequence and the sequence to be detected, said antibody reagent either being immobilized or being linked to a specifically bindable ligand, and if said binding is not immobilized, an immobilized form of a binding partner for said ligand.

MS-1362

0146039

10. The reagent system of Claim 9 wherein said antibody reagent is selective for binding (i) DNA·RNA hybrids wherein one of the probe and the sequence to be detected is DNA and the other is RNA, (ii) RNA·RNA hybrids wherein both the probe and the sequence to be detected are RNA, or (iii) intercalation complexes wherein the duplexes formed in the assay comprise a nucleic acid intercalator bound thereto in the form of intercalation complexes.

11. The reagent system of Claim 9 which additionally comprises a labeled form of a second antibody reagent which is selective for binding said duplexes, wherein the first and second antibody reagents are preferably the same substance.

12. The reagent system of Claim 9 wherein the probe comprises a label.

13. The reagent system of either of Claims 11 or 12 wherein the label is an enzymatically active group, a fluorescer, a chromophore, a luminescer, a specifically bindable ligand, or a radioisotope.

14. The reagent system of Claim 9 wherein said ligand is biotin or a hapten and said binding partner is avidin or an anti-hapten antibody, respectively.

/mc

MS-1362

COMBINE:

1. SINGLE STRANDED SAMPLE NUCLEIC ACIDS

(S)

2. POLYNUCLEOTIDE PROBE

(P)

3. IMMOBILIZED AND LABELED ANTI - HYBRID

FIG. 1

COMBINE:

I. SINGLE STRANDED SAMPLE NUCLEIC ACIDS

(S)

2. LABELED AND INTERCALATOR - MODIFIED PROBE

(P)

3. IMMOBILIZED ANTI-HYBRID AND LABELED BINDER

FIG. 2